# EUROPEAN PATENT APPLICATION

(11) **EP 2 226 082 A2**
(43) Date of publication of application: **08.09.2010**
(21) Application number: 09004943.8
(22) Date of filing: 03.04.2009
(51) Int. Cl.: A61K 49/04, A61K 49/22

(54) **Control of the toxicity of gold nanoparticles**

(30) Priority: 05.03.2009 EP 09003191
(71) Applicant: Universität Duisburg-Essen, 45141 Essen (DE)
(72) Inventor: Jahnen-Dechent, Wilhelm, Prof. Dr., 52066 Aachen (DE); Pan, Yu, 52074 Aachen (DE); Neuss-Stein, Sabine, 52249 Eschweiler (DE); Ruau, David, 52064 Aachen (DE); Simon, Ulrich, Prof. Dr., 52074 Aachen (DE); Leifert, Annika, 52064 Aachen (DE); Schmid, Günter, Prof. Dr., 42555 Velbert (DE); Brandau, Wolfgang, Prof. Dr., 44797 Bochum (DE)
(74) Representative: Gesthuysen, von Rohr & Eggert

(57) **Abstract**

The present invention relates to gold nanocluster compounds, especially gold nanoparticles, having a reduced toxicity, especially cytotoxicity, which are preferably appropriate for use in medical diagnostics such as medical imaging (e.g. X-ray, CT etc.), said gold nanocluster compounds comprising a core of at least one gold atom and at least one ligand bound to said core, wherein the reduction of toxicity, especially cytotoxicity, of said gold nanocluster compound is controlled and/or adjusted via its ligand structure and/or its ligand chemistry.

## Description

The present invention relates to gold nanocluster compounds or gold nanoparticles, respectively, and to the use thereof, especially in the fields of medicine and pharmaceuticals, medical diagnostics such as medical imaging and material sciences.

Especially, the present invention relates to gold nanocluster compounds or gold nanoparticles, respectively, having a reduced toxicity, especially cytotoxicity, as well as to their use.

Furthermore, the present invention also refers to a process of controlling and/or reducing the toxicity, especially cytotoxicity, of gold nanocluster compounds or gold nanoparticles, respectively.

In the beginning, gold nanoparticles (in the following also called "AuNPs") were generally considered to be non-toxic such as e.g. bulk gold which is inert and biocompatible. Recently, however, applicant could show that certain AuNPs, especially AuNPs of 1.4 nm diameter capped with triphenylphosphine monosulfonate (TPPMS), in the following also called "Au1.4MS", are much more cytotoxic than 15 nm nanoparticles (in the following also called "Au15MS") of similar chemical composition.

Thus, gold nanoparticles may have toxic, especially cytotoxic properties or non-toxic, especially non-cytotoxic properties, depending on the specific nature of the gold compound on which the respective gold nanoparticles are based.

Especially in the field of therapeutic applications, preferably in the treatment of tumor and cancer diseases, toxic, especially cytotoxic properties of the used metal cluster nanocompounds, especially gold nanoparticles, are desired.

Therefore, in applicant's own German application DE 102 35 602 A1 and also in the corresponding WO 2004/014401 A1 metal cluster nanocompounds, especially gold cluster nanocompounds on the basis of gold nanoparticles, for the treatment of tumor and cancer diseases are disclosed.

Furthermore, in applicant's own application EP 1 977 754 A1 toxic, especially cytotoxic gold nanocluster compounds for the treatment of tumor and cancer diseases are disclosed, wherein according to applicant's studies the toxicity, especially cytotoxicity, of the gold nanocluster compounds disclosed there is dependent on their particle size, i.e. specifically on the core size of these gold nanocluster compounds, said size ranging from 0.5 nm to 10 nm, the outer limits of this range being included.

However, especially for the purpose of medical imaging, it would be desirable to provide gold nanoparticles or gold nanocluster compounds, respectively, which do not possess any toxicity, especially cytotoxicity, in order that these compounds may be used for the purpose of medical imaging in living organisms, especially human beings.

Thus, it is an object of the present invention to provide nanoscopic gold compounds, especially gold nanocluster compounds or gold nanoparticles, respectively, which have a minimized toxicity, especially cytotoxicity, or do essentially not possess any toxicity, especially cytotoxicity, at all.

Furthermore, it is another object of the present invention to being about a reliable method or process for providing or producing nanoscopic gold compounds, especially gold nanocluster compounds or gold nanoparticles, respectively, having a reduced toxicity, especially cytotoxicity, or essentially no toxicity, especially cytotoxicity at all.

Furthermore, it is yet another object of the present invention to provide an effective method or process for modifying and/or modelling nanoscopic gold compounds, especially gold nanocluster compounds or gold nanoparticles, having reduced and/or minimized toxicity, especially cytotoxicity, in a reliable way.

Finally, it is a further object of the present invention to provide nanoscopic gold compounds, especially gold nanocluster compounds or gold nanoparticles, respectively, which are effective and/or appropriate and/or useful in diagnostic medical processes and techniques, especially in medical imaging.

Surprisingly, applicant has found out that the aforedescribed objects can be solved, *inter alia*, by the subject-matter of Claim 1; further, especially advantageous embodiments are the subject-matter of the respective dependent and independent claims.

Furthermore, according to another aspect of the present invention, the present invention refers to the use of the gold nanocluster compounds of the present invention, as defined in the respective use claims.

Finally, according to yet another aspect of the present invention, the present invention refers to a process of controlling and/or reducing the toxicity, especially cytotoxicity, of a gold nanocluster compound or gold nanoparticle, respectively, as defined in Claim 17; further, especially advantageous embodiments are the subject-matter of the respective dependent claims.

In the following, it has to be noted that explanations, details, examples etc. given with respect to one aspect of the present invention only, of course, shall also refer to all the other aspects of the present invention, even without explicit mentioning of this fact. Thus, it may well be in the following that specific aspects, explanations, details etc. are only delineated with respect to one specific embodiment only in order to avoid unnecessary repetitions, but they also apply with respect all other aspects of the present invention.

Especially, as applicant has surprisingly found out, the toxicity, especially cytotoxicity, of nanoscopic gold compounds, especially gold nanocluster compounds and/or gold nanoparticles, respectively, may be controlled and/or adjusted in an efficient and purposeful way by modelling and/or modifying and/or influencing and/or adjusting the ligand structure and/or ligand chemistry of the respective gold compound. This means that - in contrast to applicant's previous studies, according to which toxicity, especially cytotoxicity, might be controlled over the particle size or core size of the respective gold compounds - a reduced toxicity, especially cytotoxicity, may be reached or adjusted via the ligand structure or ligand chemistry.

Thus, according to a **first** aspect of the present invention, there is provided a gold nanocluster compound, especially a gold nanoparticle, having a reduced toxicity, especially cytotoxicity, preferably for use in medical diagnostics, said gold nanocluster compound comprising a core consisting of at least one gold atom and at least one ligand bound to said core, wherein the reduction of toxicity, especially cytotoxicity, of said gold nanocluster compound is controlled and/or adjusted via its ligand structure and/or ligand chemistry.

According to a specific embodiment, the gold nanocluster compound has a defined particle size, especially a defined size of the core of said gold nanocluster compound, said size ranging from 0.01 to 1,000 nm, especially from 0.1 to 100 nm, preferably from 0.5 to 50 nm, the outer limits of this range being included.

According to a first specific embodiment of the present invention, the size of the core of said gold nanocluster compound ranges from 0.5 nm to 10 nm, especially 0.8 nm to 2 nm, preferably 1.0 nm to 1.5 nm, even more preferably 1.2 nm to 1.4 nm, the outer limits of these ranges being included. Especially, according to this embodiment, the size of the core of said gold nanocluster compound is about 1.2 nm or about 1.4 nm; these core sizes are toxic, especially cytotoxic, per se, however, are rendered non-toxic, especially non-cytotoxic, by the modification/adaptation of the ligand chemistry.

According to another, alternative embodiment, the size of the core of said gold nanocluster compound is at least 10 nm, especially at least 11 nm, preferably at least 12 nm. In the case of these sizes of the cores, toxicity, especially cytotoxicity, is reduced per se if compared to smaller core sizes. However, by appropriate adaptation or variation of the ligand chemistry and/or ligand structure, the respective toxicity can be reduced even more.

In general, the gold nanocluster compound comprises gold atoms in the oxidation state of Au⁰. Usually, the core of said gold nanocluster compound comprises and/or consist of gold atoms in the oxidation state of Au⁰.

Generally, the gold nanocluster compound comprises a core comprising from 5 to 200 gold atoms, especially 20 to 80 gold atoms, preferably 25 to 70 gold atoms, more preferably 30 to 60 gold atoms, even more preferably 35 to 55 gold atoms, the outer limits of these ranges being included and the gold being preferably in the oxidation state of Au⁰.

In the case that the core size of the gold nanocluster compound is about 1.2 nm or about 1.4 nm, the gold nanocluster compound comprises a core comprising 35 gold atoms on an average or 55 gold atoms on an average, respectively, the gold being preferably in the oxidation state of Au⁰. According to this specific embodiment, the gold nanocluster compound is a Au₃₅ nanocluster compound or a Au₅₅ nanocluster compound, respectively, the gold being preferably in the oxidation state of Au⁰.

The number of ligands in the gold nanocluster compound of the present invention may vary in great ranges: Usually, the number of ligands of said gold nanocluster compound ranges from 1 to 100, especially 5 to 50, preferably 10 to 40, more preferably 12 to 35, the outer limits of these ranges being included.

In general, according to the present invention, the ligand structure and/or ligand chemistry is such that the gold nanocluster compound is stable under physiological conditions, especially if applied to a living organism, i.e. the bonding between said core and said ligand is not split or not cleaved under physiological conditions, especially not if applied to a living organism. For, applicant has found out that toxicity, especially cytotoxicity, originates from the gold core itself so that toxicity is minimized or prevented if a stable ligand structure is provided, which cannot be cleaved or split under physiological conditions (i.e. in the case of a stable gold nanocluster compound).

In order to reach the inventive objects, there are several possibilities: On the one hand, there is the possibility to provide gold nanocluster compounds which are *a priori* non-toxic, especially non-cytotoxic, due its original ligand structure/chemistry. On the other hand, there exists also the possibility to convert toxic, especially cytotoxic gold nanocluster compounds into non-toxic, especially non-cytotoxic gold nanocluster compounds, particularly either by providing a secondary outer ligand shell and/or by exchange of the ligand structure and/or ligand chemistry (i.e. in other words in general by stabilization).

As applicant has surprisingly found out, toxicity, especially cytotoxicity, of gold nanocluster compounds is mainly based on the fact that such compounds are often not stable under physiological conditions, so that ligands may be split off and the "nude" gold core (i.e. the "nude" Au⁰ core) may directly interact with the cells, thus causing (cyto-)toxicity (i.e. toxicity/cytotoxicity is thus based on the Au core alone, i.e. the Au core without ligands). Consequently, as applicant has surprisingly found out, (cyto-)toxicity of such gold nanocluster compounds may be prevented or at least reduced by their stabilization, especially by stabilization of the ligand structure and/or ligand chemistry, wherein such stabilization may be performed by several means (i.e. either by providing a secondary outer ligand shell and/or by exchange of the ligand structure and/or ligand chemistry, namely by providing a ligand structure and/or ligand chemistry which is stable under physiological conditions).

Consequently, according to a specific embodiment of the present invention, the present invention refers to a nanocluster compound, especially as defined before, wherein the gold nanocluster compound has been stabilized (i.e. rendered stable also under physiological conditions), wherein such stabilization is performed:
- by treatment with at least one antioxidant (i.e. synonymously also named as "antioxidative agent" or "reducing agent"), especially selected from the group consisting of N-acetylcysteine (NAC), glutathione (GSH), sulfonated triphenylphosphines, especially monosulfonated triphenylphosphine (TPPMS), and/or ascorbic acid as well as mixtures thereof; and/or
- by providing with a secondary ligand shell, especially wherein the secondary ligand surrounds and/or compasses said gold nanocluster compound including its core and its ligands and/or especially wherein the secondary ligand shell is provided to said gold nanocluster compound by an excess of said ligand; and/or
- by treatment with at least one sulfur-containing organic compound, especially selected from the group consisting of thiols, dithiols, disulfides, thioethers, thioesters and mixtures thereof, preferably N-acetylcysteine (NAC) and/or glutathione (GSH) or mixtures thereof.

Thus, according to a specific embodiment of the present invention, the gold nanocluster compound has been treated with at least one antioxidant (i.e. synonymously also named as "antioxidative agent" or "reducing agent"). Especially, said antioxidant may be e.g. preferably selected from the group consisting of N-acetylcysteine (NAC), glutathione (GSH), sulfonated triphenylphosphines, especially monosulfonated triphenylphosphine (TPPMS), and/or ascorbic acid as well as mixtures thereof. Such treatment reduces or even completely prevents toxicity, especially cytotoxicity.

According to another embodiment, the gold nanocluster compound has been provided with a secondary ligand shell. Especially, in this embodiment, the secondary ligand surrounds and/or compasses said gold nanocluster compound including its core and its ligands, i.e. the secondary ligand shell is provided to said gold nanocluster compound by an excess of said ligand. The expression "excess of said ligand" means that on behalf of the preparation of the respective gold nanocluster compound an excessive amount of ligand is added, i.e. a higher amount than can be bonded to the gold nanocluster core, so that - in addition to the ligands which are directly bonded to the nanocluster core - an additional secondary ligand shell is provided, which surrounds or compasses the gold nanocluster compound.

According to yet another embodiment, the gold nanocluster compound has been treated with at least one sulfur-containing organic compound, especially selected from the group consisting of thiols, dithiols, disulfides, thioethers, thioesters and mixtures thereof, preferably N-acetylcysteine (NAC) and/or glutathione (GSH) or mixtures thereof.

According to a specific embodiment, the gold nanocluster compound comprises, as said ligands, organic ligands containing at least one sulfur atom for binding to the core of said gold nanocluster compound, preferable under formation of an Au-S-bonding. Especially, the ligand may be based on or derived from organic thiols, dithiols, disulfides, thioethers, thioesters and mixtures thereof, preferably N-acetylcysteine (NAC) and/or glutathione (GSH) or mixtures thereof.

As it may be seen from the above, some substances may have a multiple stabilization function at the same time: Especially, N-acetylcysteine (NAC) and glutathione (GSH) have the effect of antioxidants, on the one hand; on the other hand, these substances are also organic ligands containing at least one sulfur atom for binding to the core of said gold nanocluster compound under formation of an Au-S-bonding, thus additionally stabilizing the gold nanocluster compound by this route. Another example is sulfonated triphenylphosphines, especially monosulfonated triphenylphosphines (TPPMS): These substances also have the effect of antioxidants, on the one hand; on the other hand, these substances, especially when used in excess amounts, provide a secondary ligand shell surrounding and/or compassing said gold nanocluster compound including its core and its ligands, i.e. a secondary ligand shell is provided to said gold nanocluster compound, thus additionally stabilizing the gold nanocluster compound via this effect.

According to a specific embodiment of the present invention, the gold nanocluster compound is represented by the general formula (I)

[Auₙ Lₘ] (I)

wherein:
- "Au" denotes the Au⁰ atoms in said gold nanocluster compound;
- "n" is a whole number denoting the number of gold atoms in said gold nanocluster compound, preferably n being selected in the range of from 20 to 80, especially 25 to 70, preferably 30 to 60, even more preferably 35 to 55, the outer limits of these ranges being included, with n being most preferably 35 if m = 35 or 55 if m = 12;
- "L", identical or different, denotes the ligand(s) in said gold nanocluster compound, especially an organic ligand, preferably a ligand based on a triphenylphosphine or a triphenylphosphine derivative, especially a sulfonated triphenylphosphine, a sulfonate, a sulfate, a phosphate, a phosphonate, a carbonate, a carboxylate or mixtures thereof, more preferably a ligand based on a triphenylphosphine or a triphenylphosphine derivative, especially a sulfonated triphenylphosphine;
- "m" is a whole number denoting the number of ligands in said gold nanocluster compound, preferably m being selected in the range of from 5 to 50, especially 10 to 40, preferably 12 to 35, the outer limits of these ranges being included, with m being most preferably 12 if n = 55 or 35 if n = 35,
wherein said gold nanocluster compound has been subjected to a stabilization treatment, wherein such stabilization treatment is performed:
- by treatment with at least one antioxidant (i.e. synonymously also named as "antioxidative agent" or "reducing agent"), especially selected from the group consisting of N-acetylcysteine (NAC), glutathione (GSH), sulfonated triphenylphosphines, especially monosulfonated triphenylphosphines (TPPMS), and/or ascorbic acid as well as mixtures thereof; and/or
- by providing with a secondary ligand shell, especially wherein the secondary ligand surrounds and/or compasses said gold nanocluster compound including its core and its ligands and/or especially wherein the secondary ligand shell is provided to said gold nanocluster compound by an excess of said ligand; and/or
- by treatment with at least one sulfur-containing organic compound, especially selected from the group consisting of thiols, dithiols, disulfides, thioethers, thioesters and mixtures thereof, preferably N-acetylcysteine (NAC) and/or glutathione (GSH) or mixtures thereof (especially wherein the sulfur-containing organic compound binds via its sulfur atom(s) to the core of the gold nanocluster compound, preferable under formation of an Au-S-bonding).

According to a specific embodiment of the present invention, the gold nanocluster compound is represented by the general formula (I)

[Auₙ Lₘ] (I)

wherein:
- "Au" denotes the Au⁰ atoms in said gold nanocluster compound;
- "n" is a whole number denoting the number of gold atoms in said gold nanocluster compound, preferably n being selected in the range of from 20 to 80, especially 25 to 70, preferably 30 to 60, even more preferably 35 to 55, the outer limits of these ranges being included, with n being most preferably 35 if m = 35 or 55 if m = 12;
- "L", identical or different, denotes the ligand(s) in said gold nanocluster compound, especially an organic ligand, preferably a ligand based on a triphenylphosphine or a triphenylphosphine derivative, especially a sulfonated triphenylphosphine, a sulfonate, a sulfate, a phosphate, a phosphonate, a carbonate, a carboxylate or mixtures thereof, more preferably a ligand based on a triphenylphosphine or a triphenylphosphine derivative, especially a sulfonated triphenylphosphine;
- "m" is a whole number denoting the number of ligands in said gold nanocluster compound, preferably m being selected in the range of from 5 to 50, especially 10 to 40, preferably 12 to 35, the outer limits of these ranges being included, with m being most preferably 12 if n = 55 or 35 if n = 35,
wherein said gold nanocluster compound has been subsequently treated with at least one antioxidant, wherein said antioxidant is preferably selected from the group consisting of N-acetylcysteine (NAC), glutathione (GSH), sulfonated triphenylphosphines, especially monosulfonated triphenylphosphine (TPPMS), and/or ascorbic acid as well as mixtures thereof.

According to another specific embodiment of the present invention, the gold nanocluster compound is represented by the general formula (I)

[Auₙ Lₘ] (I)

wherein:
- "Au" denotes the Au⁰ atoms in said gold nanocluster compound;
- "n" is a whole number denoting the number of gold atoms in said gold nanocluster compound, preferably n being selected in the range of from 20 to 80, especially 25 to 70, preferably 30 to 60, even more preferably 35 to 55, the outer limits of these ranges being included, with n being most preferably 35 if m = 35 or 55 if m = 12;
- "L", identical or different, denotes the ligand(s) in said gold nanocluster compound, especially an organic ligand, preferably a ligand based on a triphenylphosphine or a triphenylphosphine derivative, especially a sulfonated triphenylphosphine, a sulfonate, a sulfate, a phosphate, a phosphonate, a carbonate, a carboxylate or mixtures thereof, more preferably a ligand based on a triphenylphosphine or a triphenylphosphine derivative, especially a sulfonated triphenylphosphine;
- "m" is a whole number denoting the number of ligands in said gold nanocluster compound, preferably m being selected in the range of from 5 to 50, especially 10 to 40, preferably 12 to 35, the outer limits of these ranges being included, with m being most preferably 12 if n = 55 or 35 if n=35,
wherein said gold nanocluster compound has been subsequently provided with a secondary ligand shell, preferably on the basis of the same type as the ligand "L" itself, especially wherein the secondary ligand surrounds and/or compasses said gold nanocluster compound including its core and its ligands and/or especially wherein the secondary ligand shell is provided to said gold nanocluster compound by an excess of said ligand.

According to yet another specific embodiment of the present invention, the gold nanocluster compound is represented by the general formula (I)

[Auₙ Lₘ] (I)

wherein:
- "Au" denotes the Au⁰ atoms in said gold nanocluster compound;
- "n" is a whole number denoting the number of gold atoms in said gold nanocluster compound, preferably n being selected in the range of from 20 to 80, especially 25 to 70, preferably 30 to 60, even more preferably 35 to 55, the outer limits of these ranges being included, with n being most preferably 35 if m = 35 or 55 if m = 12;
- "L", identical or different, denotes the ligand(s) in said gold nanocluster compound, especially an organic ligand, preferably a ligand based on a triphenylphosphine or a triphenylphosphine derivative, especially a sulfonated triphenylphosphine, a sulfonate, a sulfate, a phosphate, a phosphonate, a carbonate, a carboxylate or mixtures thereof, more preferably a ligand based on a triphenylphosphine or a triphenylphosphine derivative, especially a sulfonated triphenylphosphine;
- "m" is a whole number denoting the number of ligands in said gold nanocluster compound, preferably m being selected in the range of from 5 to 50, especially 10 to 40, preferably 12 to 35, the outer limits of these ranges being included, with m being most preferably 12 if n = 55 or 35 if n=35,
wherein said gold nanocluster compound has been subsequently treated with at least one sulfur-containing organic compound, especially selected from the group consisting of thiols, dithiols, disulfides, thioethers, thioesters and mixtures thereof, preferably N-acetylcysteine (NAC) and/or glutathione (GSH) or mixtures thereof.

According to a further specific embodiment of the present invention, the gold nanocluster compound is represented by the general formula (I')

[Au_{n'} L'_{m'}] (I')

wherein:
- "Au" denotes the Au⁰ atoms in said gold nanocluster compound;
- "n' " is a whole number denoting the number of gold atoms in said gold nanocluster compound, preferably n' being selected in the range of from 20 to 80, especially 25 to 70, preferably 30 to 60, even more preferably 35 to 55, the outer limits of these ranges being included;
- "L' ", identical or different, denotes the ligand(s) in said gold nanocluster compound, wherein the ligand is a sulfur-containing organic ligand, especially selected from the group consisting of thiols, dithiols, disulfides, thioethers, thioesters and mixtures thereof, preferably N-acetylcysteine (NAC) and/or glutathione (GSH) or mixtures thereof;
- "m' " is a whole number denoting the number of ligands in said gold nanocluster compound, preferably m' being selected in the range of from 1 to 50, especially 1 to 40, preferably 1 to 35, the outer limits of these ranges being included.

According to a preferred embodiment, the gold nanocluster compound is water-soluble or at least dispersible in aqueous media and/or water, in particular under physiological conditions. Especially, the gold nanocluster compound possesses a water-solubility or water-dispersibility of at least 0.1 µmol/l, preferably at least 1.0 µmol/l, more preferably at least 1 mmol/l or more, and/or up to 100 mmol/l or more.

According to a **second** aspect of the present invention, there is provided a diagnostic composition, especially for the use in imaging procedures or techniques, especially in the field of human or veterinary medicine or in the field of material testing, preferably in the form of a liquid composition such as a solution or a dispersion, said diagnostic composition comprising at least one gold nanocluster compound as defined before, preferably together with at least one essentially non-toxic carrier or excipient.

The composition according to the present invention usually contains said gold nanocluster compound in diagnostically effective amounts.

Preferably, said gold nanocluster compound is dissolved or dispersed in said composition in an amount of at least 0.1 µmol/l, preferably at least 1.0 µmol/l, more preferably at least 1 mmol/l or more, and/or up to 100 mmol/l or more.

In addition, the composition of the present invention may further comprise other constituents and/or additives.

With respect to further details relating to the composition of the present invention, reference may be made to the above explanations with respect to the inventive gold nanocluster compound, which apply accordingly also to the composition of the present invention.

According to a **third** aspect of the present invention, the present invention also relates to the use of at least one gold nanocluster compound as defined before in the fields of medicine, especially human and veterinary medicine, medical technology and material sciences, especially in the testing of materials.

Furthermore, the present invention also refers of the use of at least one gold nanocluster compound as defined before in the manufacture of a diagnostic composition, especially for use in imaging procedures or imaging techniques, particularly medical imaging such as X-ray imaging, computed tomography (CT) and photoacoustic imaging or imaging in material sciences such as testing of materials.

Further, the present invention refers to the use of at least one gold nanocluster compound as defined before as a contrast agent or contrast enhancer in imaging procedures or imaging techniques, particularly medical imaging such as X-ray imaging, computed tomography (CT) or photoacoustic imaging or imaging in material sciences such as testing of materials.

With respect to the inventive use, the above explanations with respect to the inventive gold nanocluster compound and the inventive composition also apply accordingly.

Finally, according to a **fourth** aspect of the present invention, there is provided a process of controlling and/or reducing the toxicity, especially cytotoxicity, of a gold nanocluster compound or gold nanoparticle, especially a ligand-stabilized gold nanocluster compound or gold nanoparticle, preferably for use in medical diagnostics, wherein the reduction of toxicity, especially cytotoxicity, of said gold nanocluster compound or gold nanoparticle is controlled and/or adjusted via its ligand structure and/or ligand chemistry.

According to the process of the present invention, the used gold nanocluster compound or the used gold nanoparticle usually comprises a core of at least one gold atom and at least one ligand bound to said core. According to a preferred embodiment, the used gold nanocluster compound or the used nanoparticle, respectively, is as defined before.

With respect to the process of the present invention, the above explanations with respect to the inventive gold nanocluster compound, the inventive composition and the inventive use apply accordingly.

Finally, it has to be noted that, with respect to all aspects of the present invention (i.e. gold nanocluster compound of the present invention, inventive use and process of the present invention) gold may at least partially be replaced by another transition metal, especially selected from the group consisting of platinum (Pt), rhodium (Rh), iridium (Ir), palladium (Pd), ruthenium (Ru), osmium (Os) and silver (Ag) or mixtures thereof.

On the whole, as it may be concluded from the above, the present invention is based on applicant's surprising finding that the toxic, especially cytotoxic effect of ligand-stabilized metal nanocluster compounds, especially gold nanocluster compounds or gold nanoparticles, respectively, may be controlled by the design, adaptation, modification, variation etc. of the ligand chemistry and/or ligand structure. In addition, toxicity, especially cytotoxicity, can be further decreased with increasing core size of the respective nanocluster compounds. It could not be expected that the toxicity, especially cytotoxicity, of the respective chemical nanocluster compounds could be controlled via their ligand structure and/or ligand chemistry.

This offers new possibilities for such gold nanocluster compounds, especially for the fields of medicine, medical diagnostics and material sciences, especially testing of materials. Thus, the inventive compounds are useful for imaging techniques and procedures of all kinds, especially as contrast agents or contrast enhancers, as explained above.

Thus, with the present invention, significant and decisive progress could be made with respect to the control of the toxicity, especially cytotoxicity, of gold nanoparticles.

On behalf of the present invention, applicant could show that - apart from a critical size range - also ligand chemistry influences toxicity of nanoparticles decisively. Therefore, the inventive gold nanocluster compounds, which are non-toxic in nature, may be useful e.g. as contrast enhancers in medical imaging. In any case, toxicity is a prime concern for any application in living organisms. For instance, gold nanoparticles of the type Au1.4MS are cardiotoxic because the latter block hERG chanels in cardiomyocytes; however, with the inventive concept, these nanoparticles may be converted to non-toxic species via control and modification of the ligand chemistry.

Thus, the invention provides gold nanoparticles especially for medical imaging, such as X-ray, CT or photoacoustic imaging, especially as a contrast enhancer e.g. for X-ray diagnosis etc.

Since applicant has discovered what makes these small gold nanoparticles toxic and has developed the inventive concept how to prevent toxicity (i.e. e.g. by ligand exchange, e.g. with GSH instead of TPPMS etc.), this offers new possibilities for application.

According to the present invention, the toxicity of ultrasmall gold nanoparticles can be minimized to use them as contrast agents in medical imaging (e.g. gold nanoparticles substituted or treated with GSH).

The gold nanoparticles of the present invention may be used e.g. for medical imaging of blood vessels, other organs, tumors or other anatomical features. They can also be used e.g. as the only reagent, e.g. for in vivo vascular casting. Furthermore, these particles can be used for any type of clinical and/or diagnostic use.

A gold-based contrast agent or enhancer has several decisive advantages over the conventional iodine-based contrast agents: It achieves better contrast than iodine for both micro-CT and clinical CT-applications: At appropriate beam energies, gold achieves a contrast up to 3 times greater than iodine per unit mass, and initial blood contrast greater than 1,000 Hounsfield Units (HU). Furthermore, gold concentrations up to four times those of iodine can be achieved, providing a total contrast gain of up to ten times or more. Unlike iodine, a gold compound containing solution has very low viscosity and osmolality, and therefore may be injected and used in small blood vessels without risk of vascular damage. Also, gold compounds have a longer blood residence time than iodine agents. Further, gold compounds create a higher contrast, clear through kidneys, have a low toxicity, permeate angiogenic endothelium and enable the imaging of tumors.

As delineated before, in the beginning, gold nanoparticles (also called "AuNPs") were generally considered non-toxic such as bulk gold which is inert and biocompatible. Recently, however, applicant could show that AuNPs of 1.4 nm diameter capped with triphenylphosphine monosulfonate (TPPMS), also called "Au1.4MS", are much more cytotoxic than 15 nm nanoparticles (also called "Au15MS" of similar chemical composition. Here, applicant studied major cell death pathways and determined that the cytotoxicity was caused by oxidative stress. Indicators of oxidative stress, reactive oxygen species (ROS), mitochondrial potential and integrity, and mitochondrial substrate reduction were all compromised. Genome wide expression profiling using DNA gene arrays indicated robust up-regulation of stress-related genes after 6 and 12 hours incubation with 2 x IC50 concentration of Au1.4MS, but not Au15MS nanoparticles. The caspase inhibitor, Z-VAD-fmk, did not rescue the cells suggesting that necrosis, not apoptosis was the predominant pathway at this concentration.

However, surprisingly, pretreatment of these nanoparticles with antioxidants (reducing agents), e.g. N-acetylcysteine (NAC), glutathione and TPPMS, reduced the toxicity of Au1.4MS. AuNPs of similar size, but capped with glutathione (in the following also called "Au1.1GSH"), surprisingly, likewise do not induce oxidative stress.

Without being bound to any theory, applicant concludes therefrom that beside the size dependency of AuNP toxicity, ligand chemistry is a critical parameter in determining the degree of cytotoxicity. ROS generated by the AuNPs likely cause oxidative stress that is amplified by mitochondrial damage. In summary Au1.4MS nanoparticle cytotoxicity is caused by oxidative stress at multiple targets.

In general, nanomaterials are unique in that electronic, chemical and physical properties enable many promising technical and medicinal applications. It is widely accepted that nanomaterials should be thoroughly tested for health hazards or "nanotoxicity" (cf., for instance, G. Oberdörster, E. Oberdörster, J. Oberdörster, Environmental Health Perspectives 2005, 113, 823; A. Nel, T. Xia, L. Madler, N. Li, Science 2006, 311, 622; H. Fischer, W. Chan, Curr Opin Biotechnol 2007, 18, 565.). But a balanced risk analysis is currently precluded by a glaring lack of mechanistic knowledge of nanoparticle toxicity (cf. N. Lewinski, V. Colvin, R. Drezek, Small 2008, 4, 26).

In general terms, the biology of particle-induced oxidative stress is an important mechanistic paradigm on which nanomaterial toxicity can be based (cf. A. Nel, T. Xia, L. Madler, N. Li, Science 2006, 311, 622). However, nanoparticle toxicity can have multiple reasons: In the simplest case the constituent materials of nanoparticles are toxic themselves like in cadmium containing quantum dots (cf. R. Hardman, Environ Health Perspect 2006, 114, 165). Certain nanomaterials most notably titanium dioxide particles become catalytically active upon photo-activation and thus may harm cells and tissues (see W. Lee, N. Pernodet, B. Li, C. Lin, E. Hatchwell, M. Rafailovich, Chem Commun (Camb) 2007, 4815).

However, the very properties that make nanomaterials unique may also cause toxicity (see e.g. W. Jahnen-Dechent, U. Simon, Nanomed 2008, 3, 601). Small size is the most discriminating determinant mediating unique electronic, chemical, mechanical and optical properties of nanoscopic versus bulk materials. Therefore "size" is considered critical in nanomaterial toxicity. Unfortunately, variation of "size" in many studies is intermingled with variation in chemical composition, surface charge, ligand structure and chemistry as well as aspect ratio detracting from a firm conclusion that size is or is not associated with toxicity of nanomaterials. It has been shown that surface modifications of 12 nm colloidal gold nanoparticles greatly influence the cellular trajectory (see e.g. P. Nativo, I. Prior, M. Brust, ACSnano 2008, 2, 1639). Toxicity of nanoparticles also varies with their surface functionalization (see e.g. C. M. Goodman, C. D. McCusker, T. Yilmaz, V. M. Rotello, Bioconjug Chem 2004, 15, 897). However, from a certain size upward, it appears that what is actually perceived by cells is not the nanoparticle itself, but the surface associated molecules. Thus, molecules presented by the "nanoparticle scaffold" mediate biological effects when presented in a specific conformation and density (cf. e.g. W. Jiang, B. Kim, J. Rutka, W. Chan, Nat Nanotechnol 2008, 3, 145). This "nanogeometry" is a further confounder of toxicity (cf. M. Ferrari, Nat Nanotechnol 2008, 3, 131). Along these lines "shape" can also influence cellular internalization of nanomaterials (see e.g. S. Gratton, P. Ropp, P. Pohlhaus, J. Luft, V. Madden, M. Napier, J. DeSimone, Proc Natl Acad Sci U S A 2008, 105, 11613).

In summary, when particle size exceeds the dimensions of single biological macromolecules, say a globular protein, and the material acts as a scaffold attracting molecules or molecular complexes from solution, the nanomaterial itself may be chemically and biologically inert, but due to its large surface-to-volume ratio may assemble molecular complexes called the "nanoparticle corona" (cf. e.g. M. Lundqvist, J. Stigler, G. Elia, I. Lynch, T. Cedervall, K. Dawson, Proc Natl Acad Sci U S A 2008, 105, 14265) with strong biological activity.

Furthermore, the nanomaterial itself may be innocuous, but the shape or the aspect ratio may damage cells or tissues. Asbestos fibers are a showcase example for toxicity of anisotropic materials causing chronic inflammation and cancer (see e.g. C. Dostert, V. Petrilli, R. Van Bruggen, C. Steele, B. T. Mossman, J. Tschopp, Science 2008, 320, 674 ). So, carbon nanofibers with a high aspect ratio were deemed potentially harmful (see V. Kagan, H. Bayir, A. Shvedova, Nanomedicine 2005, 1, 313), although this was not confirmed in short term experiments.

Generally, it appears that nanoparticles that cannot be cleared by phagocytic cells e.g. macrophages, will "pierce" the cells and will cause chronic activation via e.g. the inflammosome molecular activation complex (see e.g. M. McDermott, J. Tschopp, Trends Mol Med 2007, 13, 381). This is one of the few examples where the target of particle interaction has been identified on the molecular level. More candidates of interaction targets may be gleaned from a recent study detailing the "bead proteome" (cf., for instance, L. Trinkle-Mulcahy, S. Boulon, Y. Lam, R. Urcia, F. Boisvert, F. Vandermoere, N. Morrice, S. Swift, U. Rothbauer, H. Leonhardt, A. Lamond, J Cell Biol 2008, 183, 223).

Thus, it remains to be demonstrated if materials of identical composition become toxic by sheer reduction in size as proved by applicant on behalf of the present invention. Applicant could show that gold nanoparticles (AuNPs) with triphenylphosphine monosulfonate (TPPMS) shells are the ideal nanomaterial to answer this question. AuNPs can be made monodisperse with distinct sizes, stability and high yield (see, for instance, L. R. Wallenberg, J. O. Bovin, G. Schmid, Surface Science 1985, 156, 256; C. Becker, T. Fries, K. Wandelt, U. Kreibig, G. Schmid, Journal of Vacuum Science & Technology B 1991, 9, 810; G. Schmid, L. F. Chi, Advanced Materials 1998, 10, 515; G. Schmid, B. Corain, European Journal of Inorganic Chemistry 2003, 3081; G. Schmid, U. Simon, Chemical Communications 2005, 697). Some of these gold clusters proved to be cytotoxic (see e.g. M. Tsoli, H. Kuhn, W. Brandau, H. Esche, G. Schmid, Small 2005, 1, 841). A diameter predominantly of 1.4 nm renders AuNPs toxic in cell cultures (cf. e.g. Y. Pan, S. Neuss, A. Leifert, M. Fischler, F. Wen, U. Simon, G. Schmid, W. Brandau, W. Jahnen-Dechent, Small 2007, 3, 1941). Sizes above up to 15 nm as well as particles smaller than 1 nm with identical core-shell chemistry were less toxic.

On behalf of the present invention, applicant could present a detailed study of the cellular response reactions toward Au1.4MS exposure. Cell response is quick and long lasting. Cells internalize the particles and mount a robust stress response on the level of membrane and mitochondria integrity and mRNA induction. Cell death predominantly by necrosis suggests strong oxidative damage and mitochondrial permeability transition as the prime cause of cell death. Applicant's observation suggests that Au1.4MS nanoparticles may produce reactive oxygen species (ROS), which apart from the mitochondrial membrane may also damage multiple targets along their cellular trajectory, including lipids of the cell membrane, components of the endocytic pathway, newly synthesized proteins, and DNA.

Surprisingly, on behalf of the present invention, applicant has discovered that the cytotoxicity of Au nanoparticles with similar size depends on the ligand chemistry.

The cytotoxicity of AuNPs depends on their size if the same ligand, especially TPPMS (i.e. triphenylphosphine monosulfonate), was used throughout. AuNPs of 1.4 nm diameter of the gold core (Au1.4MS, 55 Au atoms) are more than 100fold more toxic in terms of [Au] than 15 nm particles consisting of identical constituents (also called "Au15MS"). Here, appli-applicant could show on behalf of the present invention that the ligand chemistry influences the cytotoxicity of ultrasmall AuNPs.

To this end, applicant incubated HeLa cervix carcinoma epithelial cells with Au1.4MS and AuNPs of similar size, but with glutathione ligand ("Au1.1GSH"). Applicant treated the cells in their logarithmic growth phase when they are most vulnerable to toxic effects. Applicant studied the cell response by vitality assays using MTT, flow cytometry and fluorescence microscopy using pathway-specific dyes as well by gene expression analysis using DNA gene arrays.

The figures illustrate the present invention, however, without limiting the invention.

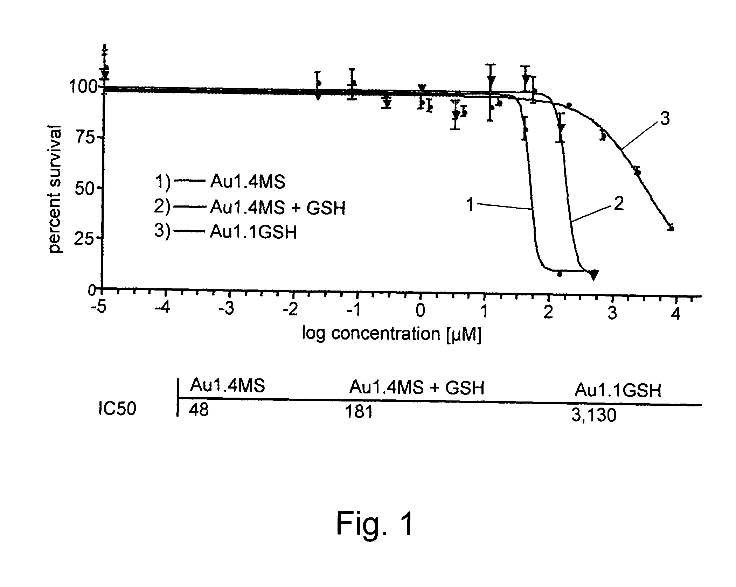

- Figure 1: IC50 of Au1.4MS and Au1.1GSH treated HeLa cells. Au1.1GSH shown in curve 3 (IC50 =3130 µM) has a 65fold higher IC50 than Au1.4MS shown in curve 1 (IC50 =48 µM). The IC50 of Au1.4MS admixed with 10 eq. of GSH was intermediary at 181 µM (curve2).

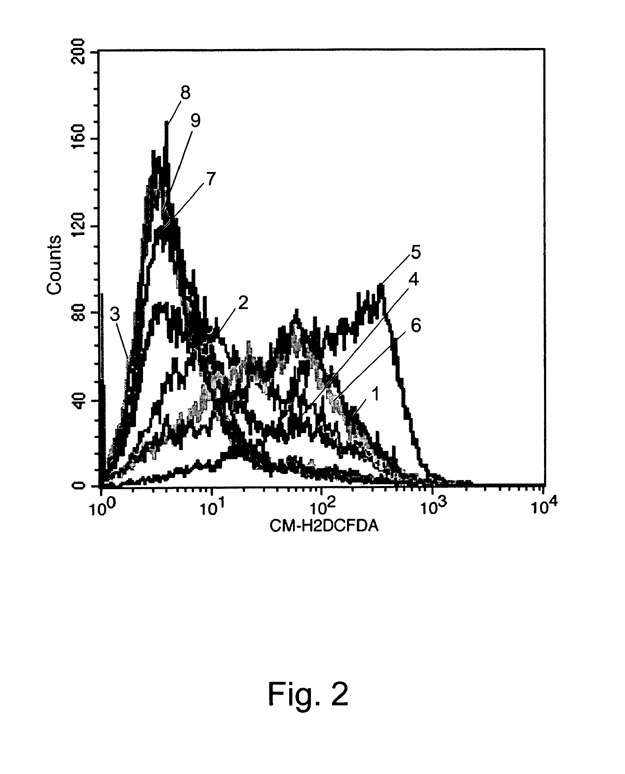

- Figure 2: Flow cytometry determination of oxidative stress. CM-H2DCFDA staining shows that Au1.4MS but not Au15MS and Au1.1GSH induced oxidative stress in HeLa. Curve 3 represented the untreated HeLa cells showing no oxidative stress. The curve 5 represents HeLa cells treated with 0.3 % H₂O₂ for 30 minutes suffering strong oxidative stress. HeLa cells treated with 100 µM Au1.4MS for 6, 12, 18, 24, and 48 hours, respectively curve 7, curve 4 curve 2, curve 6, and curve 1 showed progressively increasing accumulation of intracellular fluorescein and thus oxidative stress. The oxidative stress induced by Au1.4MS was obvious after 12 hours (curve 4) and steadily increased until the end of the test at 48 hours. In contrast HeLa cells treated for 48 hours with 1000 µM 15 nm Au15MS (curve 8) or 1000 µM Au1.1GSH (curve 9) showed no elevated intracellular fluorescein and thus no oxidative stress even at 10fold higher concentration than Au1.4MS.

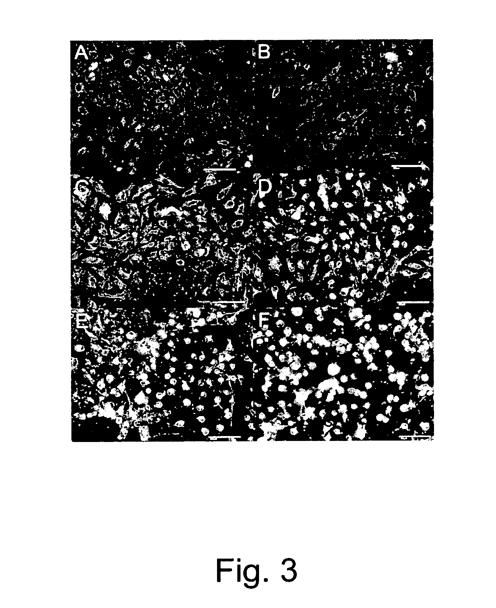

- Figure 3: Fluorescent mitochondria potential staining. JC-1 staining indicated mitochondrial depolarization after incubation with Au1.4MS. HeLa cells were incubated with 100 µM Au1.4MS and stained with the fluorescent stain JC-1. Dark punctuated staining indicated aggregation of JC-1 in intact mitochondria. Bright staining of the cytoplasm indicated mitochondrial permeability transition, PT and depolarization with concomitant discharge of JC-1 monomer into the cytoplasm. A) untreated HeLa cells, B) 1 hour treatment with 100 µM Au1.4MS. Further incubation for C) 6 hours, D) 12 hours, E) 18 hours F) 24 hours showed progressive and continued mitochondrial PT.

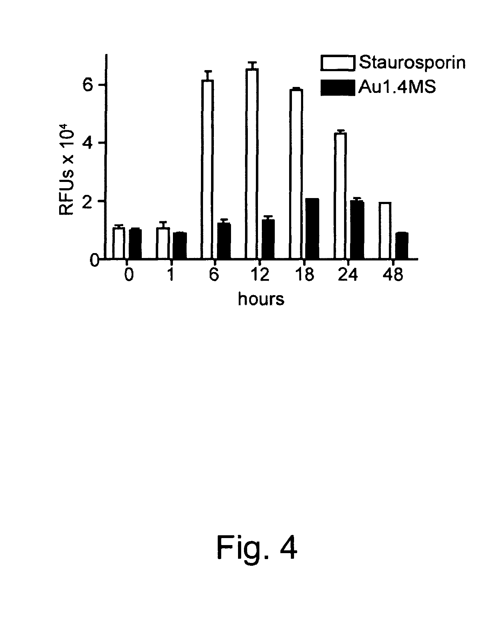

- Figure 4: Fluorogenic caspase activity determination. Caspase 3/7 activity increased 6.5fold in staurosporine treated HeLa but only 2fold in Au1.4MS treated cells. Caspase 3/7 activity was measured using a fluorogenic protease substrate and is presented as relative fluorescence unit (RFU). Treatment with 0.2 µM staurosporine for 6-18 hours strongly enhanced caspase 3/7 after incubating the cells for 6 hours and the content of caspase 3/7 reached the peak after 12 hours incubation and then decreased with the further longer incubation. 50 µM Au1.4MS treated cells has low caspase 3/7 activity after cells were incubated with Au1.4MS for 18 and 24 hours.

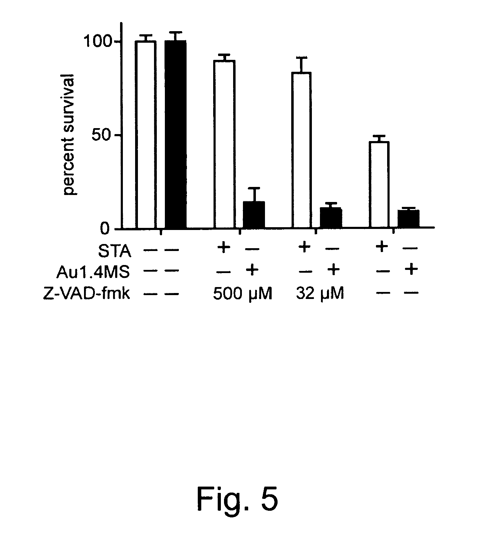

- Figure 5: Reversal of apoptosis by caspase inhibition. The caspase inhibitor Z-VAD-fmk inhibited staurosporin-triggered apoptosis, but not Au1.4MS-triggered necrosis. HeLa cells were left untreated or treated with the caspase inhibitor, Z-VAD-fmk or with staurosporine (STA) and Au1.4MS as indicated. Z-VAD-fmk inhibited cell death in staurosporin-treated cells suggesting apoptosis as the predominant death pathway. When HeLa cells were incubated with staurosporine alone, 47 % cells survived after 48 hours. The addition of Z-VAD-fmk increased survival to 90 % and 84 %, respectively. Z-VAD-fmk did not increase the survival of Au1.4MS treated cells suggesting that necrosis was the predominant death pathway.

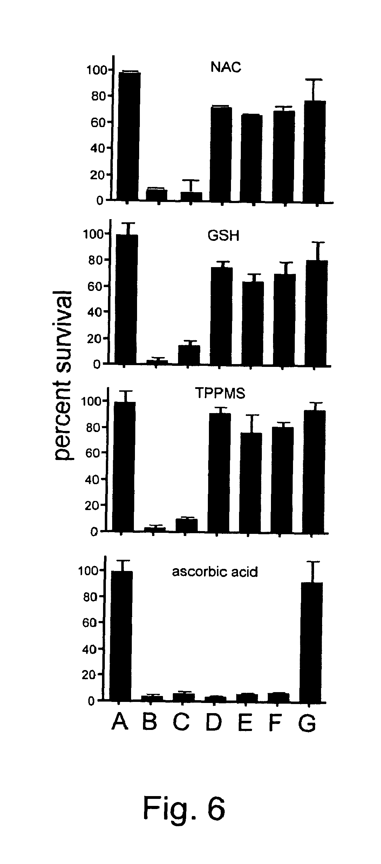

- Figure 6: NAC, GSH and TPPMS but not ascorbic acid can partially inhibit the cytotoxicity of 100 µM Au1.4MS. A) untreated cells. B) cells treated with Au1.4MS for 48 hours. C) cells pretreated with antioxidant/reducing agent for 3 hours, washed and post-treated with Au1.4MS for 48 hours. D) Au1.4MS pre-treated with antioxidant/reducing agent for 3 hours, mixture added to cells for 48 hours. E) cells pre-treated with antioxidant/reducing agent for 3 hours, then added Au1.4MS and incubated for 48 hours. F) Antioxidant/reducing agent mixed with Au1.4MS and mixture immediately added to cells and incubated for 48 hours. G) cells incuba- ted with antioxidant/reducing agent for 48 hours. N=3 in all cases; *P*<0.001 for B/D, B/E, B/F comparisons determined by ANOVA.

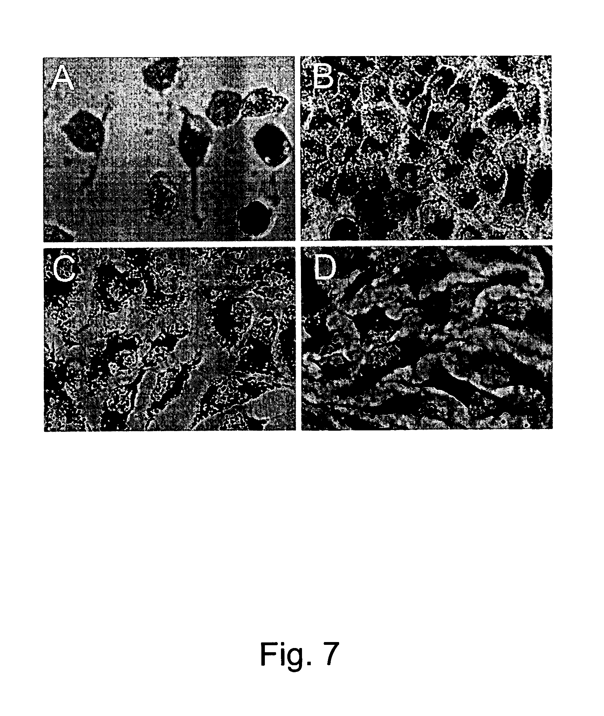

- Figure 7: Viability test for intact mitochondria and respiratory activity. (A) Cells were treated for 48 hours with Au1.4MS, (B) a mixture of Au1.4MS and GSH, (C) Au1.1GSH and (D) GSH alone. MTT was added to the cells for 2 hours to measure respiratory activity and thus viability as the amount of MTT reduced to formazan. Mitochondria stained dark due to formazan accumulation. Please note that mixing Au1.4MS greatly reduced the toxicity (A, C) and that Au1.1GSH and GSH were nontoxic to begin with.

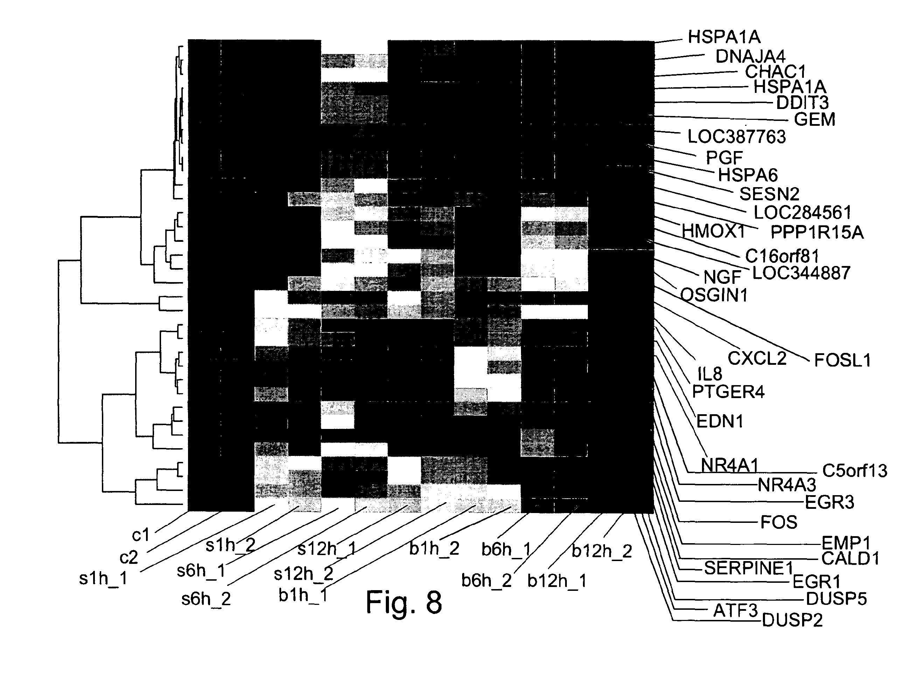

- Figure 8: Hierarchical cluster analysis and heat map representation of differentially regulated genes in AuNP treated HeLa cells. All gene chip analyses were performed in duplicates (_1, _2). HeLa cells were left untreated (c) or were treated for 1, 6 and 12 hours with Au1.4MS (s1h-s12h or small AuNP) or with Au15MS (b1h-b12h for big AuNP). Gene expression levels determined by Affymetrix^{®} gene chips were subjected to hierarchical cluster analysis. Upon treatment with Au1.4MS, 35 genes were significantly up-regulated. Each gene is depicted by a single row of colored boxes. The contrast or intensity of the respective box in one row represents the expression value of the gene transcript in one sample compared with the median expression level of the gene's transcript for all samples shown.

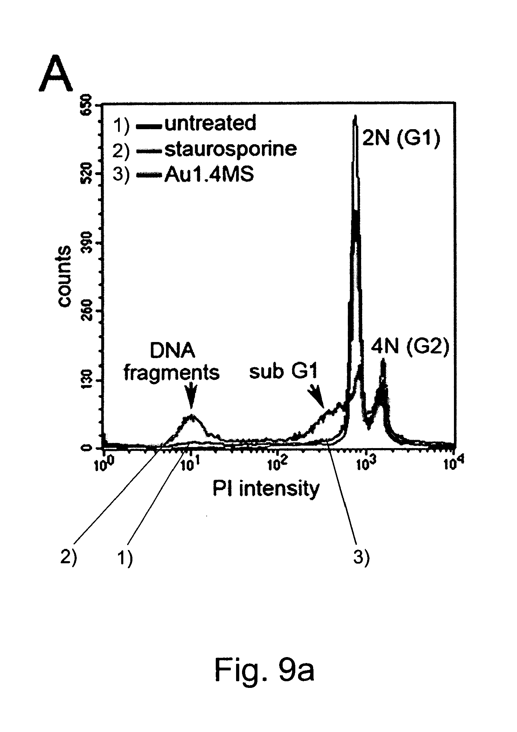

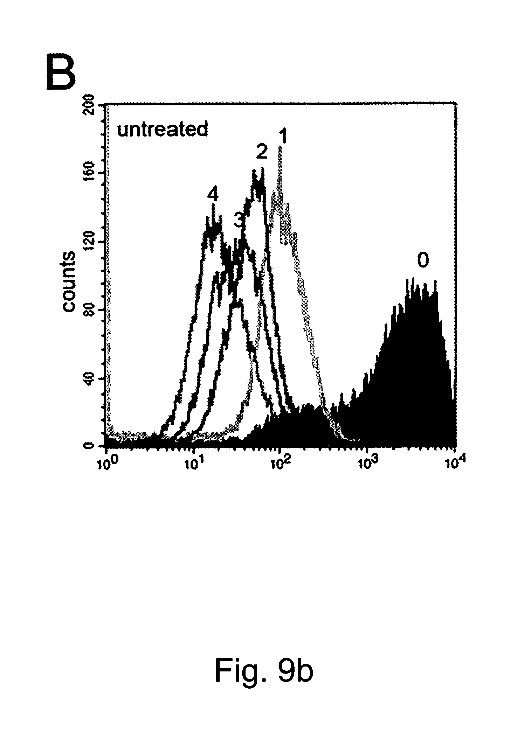

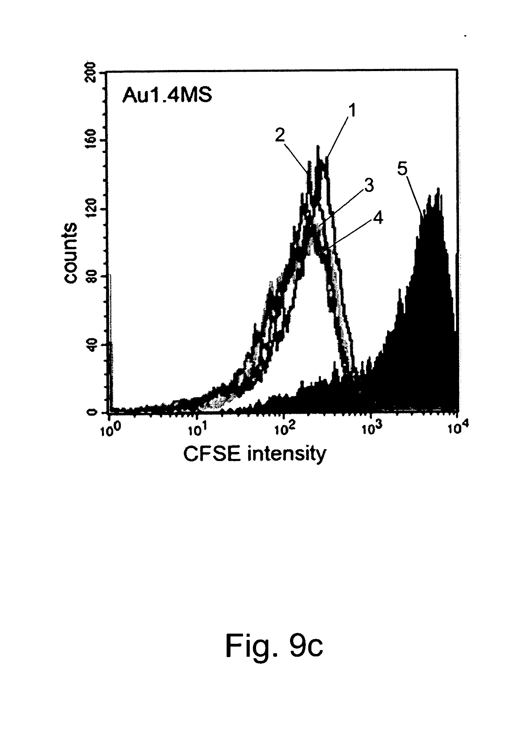

- Figure 9: Flow cytometry of DNA content and cell division. (A) Cells were labeled with propidium iodide (PI) and analysed by flow cytometry. Cells treated with staurosporine showed a hypodiploid peak in DNA content typical of G2/M arrest and apoptosis (arrow head) while untreated cells and cells treated with Au1.4MS showed normal DNA content. (B) Cells were labelled with the fluorescent dye, CFSE and further grown for 0, 1, 2, 3, and 4 days. Untreated cells (top panel) underwent 4 cell divisions with a concomitant decrease in CFSE fluorescence intensity. Cells treated with Au1.4MS divided only once, i.e. never entered a fresh cell cycle.

Figure 1 shows that the IC50 of Au1.1GSH was 3130 µM (Figure 1, curve 3), thus 65fold higher than the IC50 of Au 1.4MS, which was 48 µM (Figure 1, curve 1). When applicant mixed Au1.4MS and GSH (10 eq) and added the mixture to the cells (Figure 1, curve 2), the IC50 of the mixture was 181 µM and thus almost 4fold higher than Au1.4MS alone. This result can be interpreted in two ways. Either, excess GSH could have replaced TPPMS as the ligand due to stronger Au-S bond as compared to the Au-P bond in the starting compound, effectively creating Au1.4GSH. To further investigate this, the mixture of Au1.4MS and GSH was characterized by different means (see Experimental Section for details). The particles showed amphoteric behavior, i.e. they were well soluble in acidic and basic solution. Zeta potential measurements gave -48 mV in basic and +25 mV in acidic solution, compared to a value of -42 mV for Au1.4MS in bidistilled water at pH 7, due to the acidity of the sulfonate group. A ³¹P NMR spectrum of the washed reaction product showed no signal at all, whereas an IR spectrum in KBr showed typical signals of GSH, with the absence of the S-H stretching vibration at 2526 cm⁻¹, indicating that the GSH was attached covalently to the gold surface via the SH-group (cf. M. Habeeb Mohammed, T. Pradeep, Chemical Physics Letters 2007, 449, 186). The transmission electron microscopy (TEM) micrographs display a slightly broadened size distribution after ligand exchange but still with a mean particle size of 1.4 nm. Thus the Au1.4MS nanoparticles had exchanged GSH ligand for TPPMS.

Alternatively, Au1.4MS may have caused oxidative stress in the cells that was diminished in Au1.4MS + GSH or Au 1.1 GSH which contain thiols and are thus intrinsically antioxidant.

Applicant also studied the oxidative stress and mitochondrial damage induced by Au1.4MS nanoparticles. Applicant employed the cell permeable stain CM-H2DCFDA, which becomes fluorescent upon oxidation by intracellular reactive oxygen species, ROS, to directly demonstrate intracellular ROS by flow cytometry. Applicant used 0.3 % H₂O₂ as a positive control. Figure 2 shows the flow cytometry analysis of HeLa cells that were left untreated and showed no fluorescence (curve 3) indicating no oxidative stress. Upon treatment with 0.3 % H₂O₂ a marked right shift towards stronger fluorescence indicated the formation of fluorescein and thus strong ROS generation in H₂O₂-treated He-La cells (curve 5). ROS was likewise detected in HeLa cells treated with 100 µM Au1.4MS for 12 hours (curve 4). Intracellular ROS content and thus fluorescence intensity continuously increased until the end of the test at 48 hours. In contrast, both Au15MS (curve 8) and Au1.1GSH (curve 9) did not trigger the formation of intracellular ROS even at 10fold higher concentration. Treatment of the cells with TPPMS alone also did not cause an increase in intracellular fluorescence (not shown).

Oxidative stress is associated with protein and lipid oxidation ultimately leading to a profound alteration in mitochondrial function thought to constitute the central executioner of cell death (cf. G. Kroemer, Cell Death Differ 1997, 4, 443). A salient feature of mitochondrial damage is the mitochondrial permeability transition (PT), which results in a sudden increase in the permeability of inner mitochondrial membrane to solutes < 1500 Da. This leakiness is readily monitored with the fluorescent dye JC-1, which accumulates along the mitochondrial potential (Δψ) in mitochondria of healthy cells. At high concentration JC-1 dimerizes and fluoresces red. Upon PT the mitochondria become leaky and release JC-1 into the cytoplasm where it fluoresces green as a monomer. Figure 3A shows that untreated HeLa cells with polarized mitochondria stained dark. Treating the cells with Au1.4MS for 1, 6, 12, 18 and 24 hours caused progressive rounding up of the cells, loss of JC-1 dimers (dark) in mitochondria and discharge of monomeric JC-1 (bright) into the cytoplasm (Figure 3B-F). This observation indicates that PT and thus green cytoplasma fluorescence continuously increased up to 24 hours (shown as bright spots in Figure 3F) at which time most of the HeLa stained green and thus positive for PT.

On behalf of the present invention, applicant could show that Au1.4MS nanoparticles kill cells by necrosis. When PT is induced in a massive way and lack of mitochondrial activity rapidly depletes the cellular ATP supply, necrosis occurs, i.e. the primary disruption of the plasma membrane. When PT occurs gradually, apoptogenic proteases are activated and can act on nuclear and cytoplasmic substrates to execute apoptosis. This explains why many compounds including Au1.4MS (see e.g. Y. Pan, S. Neuss, A. Leifert, M. Fischler, F. Wen, U. Simon, G. Schmid, W. Brandau, W. Jahnen-Dechent, Small 2007, 3, 1941) induce necrosis at high doses and apoptosis at lower, subnecrotic doses (see e.g. G. Kroemer, Adv Immunol 1995, 58, 211).

To confirm that the cells underwent necrosis instead of apoptosis applicant measured the caspase 3/7 activity (Figure 4) using the fluorogenic substrate rhodamine 110 bis-(N-CBZ-L-aspartyl-L-glutamyl-L-valyl-aspartic acid amide), Z-DEVD-R110. Caspase 3/7 enzymes cleave carboxy-terminal to the aspartate in the DEVD peptide thus converting the substrate into fluorescent rhodamine. Figure 4 shows that staurosporine, a prototypic inducer of apoptosis, increased intracellular caspase 3/7 activity 6fold over background within 6 hours and reached peak activity after 12 hours. Thereafter caspase 3/7 activity declined until the end of the experiment at 48 hours. In contrast Au1.4MS treated HeLa cells showed a comparatively small caspase 3/7 induction of 2fold peaking at 18 hours.

To test if the increase in caspase 3/7 activity was necessary and sufficient to trigger cell death, applicant employed the caspase inhibitor Z-VAD-fmk. Apoptosis is predominantly caspase-mediated and can be blocked by Z-VAD-fmk whereas necrosis cannot. Figure 5 shows that the survival rate of staurosporin-treated HeLa cells (white bars) increased from 47 % to 90 % and 83 % after adding 500 µM and 32 µM Z-VAD-fmk, respectively. In contrast Z-VAD-fmk treatment did not increase cell survival in Au1.4MS treated HeLa cells again suggesting that (secondary) necrosis not apoptosis occurred in these cells.

Applicant also studied the origin of oxidative stress in Au1.4MS treated cells. From the results presented above, applicant concluded that Au1.4MS nanoparticles induced oxidative stress, caused mitochondrial permeability transition and triggered cell death by necrosis. Next, applicant asked if the oxidative stress was caused by ROS emanating from the AuNPs themselves or if ROS production occurred secondary to AuNPs endocytosis and interaction with intracellular target molecules that triggered an oxidative burst reaction in the cells. To this end, applicant pretreated the Aul.4MS nanoparticles or the cells with the antioxidants/reducing agents N-acetylcysteine (NAC), glutathione, TPPMS and ascorbic acid and studied, if a specific treatment could abolish the Au1.4MS toxicity. Figure 6 shows the cell survival of untreated HeLa cells (Figure 6 columns A), cells treated with 100 µM Au 1.4MS alone (columns B) or in combination. Treatment with Aul.4MS alone killed 96 % of cells within 48 hours. Pre-treatment of the cells (columns C) with antioxidants/reducing agents NAC, GSH, TPPMS and ascorbic acid effected a slight increase in cell survival to 7 %, 15 %, 11 % and 6 %, respectively. In contrast pre-treatment and co-incubation of Au1.4MS with NAC, glutathione and TPPMS restored cell survival to values between 65 % and 93 % of the untreated cells (Figure 6, columns D-F). Thus, treating HeLa cells with antioxidants/reducing agents alone hardly influenced cell survival while co-incubation of Au1.4MS with antioxidants/reducing agents NAC, GSH or TPPMS, but not ascorbic acid for the entire duration of the experiment irrespective of the sequence of mixing the reagents increased cell survival considerably. This suggested that the toxicity occurred not once, but continuously during cell culture. The fact that NAC, GSH and TPPMS, but not ascorbic acid were able to reduce toxicity suggested that thiol-containing compounds or an excess of the authentic ligand, but not a mere antioxidant could neutralize Au1.4MS interaction with vital biological targets or by toxic compounds emanating from Au1.4MS activity. This latter alternative, neutralization of toxic reactants is strongly supported by the finding that nanoparticles in general create ROS from dioxygen due to their high surface/volume ratio and the specific electronic configuration of the surface Au atoms (cf. A. Nel, T. Xia, L. Madler, N. Li, Science 2006, 311, 622) and furthermore that the closely related gold-55 cluster compound, Au1.4TPP (TPP: triphenyl phosphine), which is soluble in organic solvents was shown to selectively oxidize with dioxygen both in gaseous phase (see M. Turner, V. Golovko, O. Vaughan, P. Abdulkin, A. Berenguer-Murcia, M. Tikhov, B. Johnson, R. Lambert, Nature 2008, 454, 981) and in solution (see P. Ionita, M. Conte, B. Gilbert, V. Chechik, Org Biomol Chem 2007, 5, 3504). Gold-55 clusters are also remarkably resistant to oxidation rendering them ef fective oxidation catalysts most likely due to the closed-shell structure of magic number clusters (see H. Boyen, G. Kastle, F. Weigl, B. Koslowski, C. Dietrich, P. Ziemann, J. Spatz, S. Riethmuller, C. Hartmann, M. M61ler, G. Schmid, M. Garnier, P. Oelhafen, Science 2002, 297, 1533). Surprisingly, thiol-capped gold nanoparticles were inactive in similar free radical oxidations and halogen abstractions most likely due to the stronger ligand-to-metal bond (see P. Ionita, M. Conte, B. Gilbert, V. Chechik, Org Biomol Chem 2007, 5, 3504) of GSH sulfur to the surface of the AuNPs. This may explain why the thiol containing antioxidants NAC and GSH could neutralize the toxicity of Aul.4MS while the non-thiol containing antioxidant ascorbic acid could not (Figure 6). Nevertheless partial ligand exchange by NAC and GSH as well as preventing Au1.4MS interaction of the gold core with biological targets by an excess of TPPMS as the leaving group remain viable alternatives reducing Aul.4MS toxicity. In summary neutralization of continuously formed ROS by antioxidants as well as passivation of Au1.4MS by thiol-containing antioxidants are paradigms explaining why Aul.4MS was toxic, mixtures of Aul.4MS and reducing ligands were less toxic and fully GSH-capped AuNPs were least toxic in HeLa cells (Figure 1).

Figure 7 shows microscopic views of cells treated with Aul.4MS demonstrating that most of the cells died and lost the mitochondria function after treatment with 100 µM Au1.4MS (Figure 7A). When the cells were treated with a mixture of Au1.4MS and glutathione, most of the cells retained normal morphology and mitochondria activity (Figure 7B). As a note of caution, it has to be noted that NAC, GSH, TPPMS and ascorbic acid were all able to reduce the vital dye MTT into formazan in solution. Careful rinsing of the cells before the addition of MTT effectively excluded this extracellular reaction, which may produce erroneously high survival scores if it goes unnoticed. Thus, routine microscopic observation of cells to demonstrate that MTT was converted to formazan only in mitochondria, not in the cytoplasm or outside the cells is strongly advised in this kind of assay.

Furthermore, applicant could show that stress-related and inflammation-related genes are up-regulated and cell cycle related genes are down-regulated in Au 1.4MS treated cells. Having established that Au 1.4MS was cytotoxic because of continuous generation of ROS, applicant also showed that the oxidative stress would be reflected on the level of gene expression. To this end, applicant performed genome-wide mRNA expression analysis using Affymetrix^{®} gene chips. mRNA was extracted from untreated, 100 µM Au1.4MS and 1000 µM Au15MS treated cells after defined incubation periods and reverse transcribed into cDNA. The level of GAPDH house keeping gene was independently measured by RT-PCR to ensure that equal amounts of cDNA entered the analysis (not shown). Our previous studies of AuNP interaction with DNA (see Y. Liu, W. Meyer-Zaika, S. Franzka, G. Schmid, M. Tsoli, H. Kuhn, Angew Chem Int Ed Engl 2003, 42, 2853; M. Tsoli, Universität Duisburg-Essen (Essen), **2004**) had suggested that the toxicity of Au1.4MS might be due to interference with DNA transcription. However, the strongly enhanced expression of 35 genes after exposure of HeLa cells to Au1.4MS and the continued expression of GAPDH both argued against direct transcriptional inhibition by Au1.4MS.

Figure 8 shows that a group of growth related genes (PTGER4, EDN1, NR4A1, C5orf13, NR4A3, EGR3, FOS, EMP1, CALD1, SERPINE1, EGR1, DUSP5, ATF3, DUSP2) were up-regulated in both HeLa cells treated with Au1.4MS and with Au15MS at one hour after the onset of treatments (s1h_1. s1h_2, b1h_1, b1h_2). This reflected an initial growth response triggered by addition of fresh media along with the Au1.4MS and Au15MS illustrating a well-known short-term phenomenon of cell culture and confirming the validity of the gene chip expression study. A separate clustering of the gene expression changes following treatment with the non-toxic Au15MS, which confirmed an overlapping, almost identical group of genes (EGR1, NR4A1, DUSP5, PPP1R3B, EDN1, FOS, EGR1, EDN1, ADAMTS1, ATF3, PTGER4, CYR61) as up-regulated at 1 hour after medium exchange irrespective of toxicity (not shown). Following the initial growth response heat shock and stress-related genes were up-regulated after 6 hours and strongly up-regulated after 12 hours in Au1.4MS treated, but not in Au15MS or in untreated HeLa cells. This group of genes (HSPA1A, DNAJA4, CHAC1, HSPA1A, DDIT3, GEM, LOC387763, PGF, HSPA6, SESN2, LOC284561, PPP1R15A, HMOX1, C16orf81, LOC344887, NGF, OSGIN1, FOSL1, CXCL2, IL8) suggested that a robust stress response had occurred in the Au1.4MS treated cells. Highly elevated expression of heat shock proteins has been demonstrated to inhibit apoptosis at several stages including blocking of cytochrome C release from mitochondria, preventing the formation of an apoptosome and the activation of caspase-3 (see D. Mosser, R. Morimoto, Oncogene 2004, 23, 2907) ultimately forcing cells into necrosis instead of apoptosis.

Taken together the gene expression profile in Au1.4MS is fully compatible with an oxidative stress response leading to necrosis. In addition, oxidative stress and inflammation related genes including glutathione-S transferase (GST), heme oxygenase-1 (HMOX1), oxidative stress induced growth inhibit (OSGIN1) and IL-8 were also up-regulated. Most of the down-regulated genes are associated with the cell cycle including MEF2C, CCNG2, CCNE2, BRIP1, CCNE1, BARDI, CCNJ, CDKN2C, FBX04, CDKN2B (data not shown). In summary this finding suggested that continuous ROS generation was indeed the toxicity mechanism causing cell damage. Early repair reactions were started, but down-regulation of the cell cycle associated genes show that eventually secondary necrosis ensued, most likely because the cells were unable to repair the sustained multi-target damage.

Furthermore, applicant has found that it is not the oxidative stress toxicity nor the changes in gene expression that bring about a specific block in the cell cycle due to interference with e.g. DNA replication but rather that cells simply stop dividing because they die of continued oxidative stress and secondary necrosis. The protein p53 controls both the G2/M and the G1 cell cycle checkpoints and mediates reversible growth arrest in apoptotic fibroblasts (cf. M. Agarwal, A. Agarwal, W. Taylor, G. Stark, Proc Natl Acad Sci U S A 1995, 92, 8493). To gain insight into the cell cycle progression in the context of Aul.4MS cytotoxicity, applicant measured the cellular DNA content and mitotic index of HeLa cells. Figure 9A shows the result of a typical flow cytometry DNA content measurement of HeLa cells. Untreated cells (curve 1) showed a minor peak of propidium iodide (PI) fluorescence at 1.5 x 10³ depicting 4N cellular DNA content (G2 phase, about 20 % of cells) and a major peak of fluorescence at 8 x 10² indicating 2N cellular DNA content (Gl phase, 70 % of the cells). Cells staining intermediary reside in S phase. When the HeLa cells were treated with staurosporine (curve 2) to trigger apoptosis the relative proportion of cells in G2 phase increased to 50 % indicating a G2/M block of the cells. Together with the sub G1 peak and the low fluorescent peak indicating DNA fragments this pattern was typical of apoptosis. Unlike the staurosporine treated cells, Aul.4MS treated cells (curve 3) showed no G2/M block, no sub G or fragmented DNA peaks, suggesting that these cells did not execute the late stages of apoptosis including DNA fragmentation, but went straight into necrosis once the mitochondrial damage was done and PT had occurred. This is further corroborated by the fact that caspase activation, which is likewise a late event in apoptosis, but not in necrosis, was low in Aul.4MS treated cells and furthermore, that caspase inhibition by Z-VADfmk enhanced cell viability in staurosporine treated (apoptotic) cells, but not in Aul.4MS treated (secondary necrotic) cells (Figure 4, 5).

On the whole, the following conclusion may be drawn from applicant's studies: Applicant has previously shown that triphenyl phosphine sulfonate capped gold-55 clusters with a diameter of the gold core of 1.4 nm (Aul.4MS) were more toxic than smaller or larger AuNPs with similar chemical composition. Dose-dependently these compounds effected secondary necrosis. Here, applicant extends this finding in that AuNPs with similar size yet different ligand capping with GSH are markedly less cytotoxic. The toxicity profile of small AuNPs strikingly resembled their catalytic properties in gas phase or organic phase oxidation and halogen abstraction reactions. Applicant therefore concludes that the toxicity of small AuNPs depends on their ability to trigger the intracellular formation of reactive oxygen species, ROS from dioxygen. The cellular responses observed after AuNP exposure indicated a strong oxidative stress response that exacerbated cellular ROS. The fact that antioxidants reduced toxicity and that the cells executed a strong genomic stress response both support this concept.

Further embodiments, modifications and variations of the present invention are obvious to the skilled practitioner by reading the present specification and/or can be implemented by him without leaving the scope of the present invention.

The present invention is illustrated on the basis of the following exemplary embodiments which, however, do not limit the present invention in any way.

### EXPERIMENTAL SECTION

### Gold Nanoparticle Synthesis

AuPPh₃CI, benzene, BF₃-OEt₂, Chic12, diethylene glycol dimethyl ether, ethanol, HAuCl₄·3H₂O, H₂S0₄, NaBH₄, PPh₃ and sodium citrate dihydrate were purchased from diverse suppliers at the highest purity available. All chemicals were used as received, and H₂P was obtained from a Purelab Plus^{®} water purification system. TPPMS was synthesized as described F. Joó, J. Kovács, Á. Kathó, A. Bényei, T. Decuir, D. Darensbourg, A. Miedaner, D. Dubois, Inorg. Synth. 1998, 32, 1.

Au1.4MS and Au15MS were synthesized as described (Y. Pan, S. Neuss, A. Leifert, M. Fischler, F. Wen, U. Simon, G. Schmid, W. Brandau, W. Jahnen-Dechent, Small 2007, 3, 1941). Au1.1GSH was synthesized according to a published protocol (Y. Negishi, Y. Takasugi, S. Sato, H. Yao, K. Kimura, T. Tsukuda, J Am Chem Soc 2004, 126, 6518). Briefly, 100 mg (0.25 mmol) of HAuCl₄ were dissolved in 50 mL methanol. 154 mg of GSH were added. The solution was cooled to 0 °C. 12.5 mL of a freshly prepared 0.2 M solution of NaBH₄ was added dropwise over a period of five minutes, in which the color of the solution changed from yellow to dark brown. The reaction mixture was stirred for 30 minutes and the formed dark brown precipitate was centrifuged. After consecutive washing with a H₂O/methanol mixture (1 : 10, V : V) and pure methanol, the solid was dissolved in H₂O and filtered through a Milipore^{®} filter (pore diameter 20 nm). The H₂O was removed and the product was stored in solid form. The mean particle size was determined by TEM (FEI Titan S). The sample was prepared by adding 5 µL of a diluted solution onto a carbon-coated copper-grid (Figure S1 in Supporting Information).

Based on elemental analysis the number of ligands per nanoparticle has been determined. For Au1.4MS, applicant found a Au : MS ratio of 55 : 12, while for Au1.1GSH a Au : GSH ratio of 28 : 11 was derived.

### Characterization of Reaction Mixture of Au1.4MS and GSH

For the chemical analysis of the reaction product of Au1.4MS and GSH, the reaction had to be conducted in higher concentrations than for the cell tests but with the same ratio of Au1.4MS and GSH. It was noticeable that in this case the particles became less soluble in bidistilled water after an incubation time of 3 h at 37 °C. The particles could easily be centrifuged and washed several times with water. The remaining residue could be dissolved in either basic (NaOH) or acidic (HCl) solution, a first hint that the amphoteric GSH could have replaced the TPPMS on the particle surface. A ³¹P NMR of the washed product in acidic D₂O showed no signal at all, at a comparable concentration and measuring condition than a sample of Au1.4MS which showed a signal at 36.5 ppm (Varian Mercury 200). Furthermore, the zeta potentials of the basic and acidic solutions were determined to -48 mV and +25 mV, respectively, which corresponds well to the functionalities of GSH (one free amine group that can be protonated by HCl versus two free carboxylic acid groups which may be deprotonated under basic conditions). The zeta potential of Au1.4MS in bidistilled water at pH 7 was -42 mV, due to the acidity of the sulfonate group (Malvern Zeta-Sizer). Moreover, an IR spectrum of the reaction product in a KBr pellet was recorded (Bruker Vertex 70, (Figure S3 in Supporting Information)). A TEM sample was prepared as explained above (data shown in figure S2 in Supporting Information).

### Cell Culture and Cytotoxicity Assays

HeLa human cervix carcinoma cells were cultured in DMEM low glucose medium. Media contained 10 % fetal calf serum, 2.9 mg/mL L-glutamine, 1 mg/mL streptomycin and 1000 units/mL penicillin. All cells were cultured at 37 °C in water saturated air supplemented with 5 % CO₂. Culture media were changed every three days. Cells were passaged once a week. Cell numbers were estimated using a cell counter (Schaerfe cell counting system, Germany).

Cells were plated in 96 well microtiter plates at initial densities of 2000 cells per well. Cell culture medium was changed every three days. The cell growth was tested by the colorimetric MTT assay, which measures the conversion of the yellowish water soluble tetrazolium salt to a water-insoluble purple formazan product within viable breathing cells as a proxy of cell number and viability. The water insoluble formazan was dissolved in 100 µL of a solvent mixture consisting of 80 µL isopropanol with 0.04 µM hydrochloride acid and 20 µL 3 % SDS. Absorption of the samples was measured with a spectrophotometer at 584 nm. The amount of formazan produced is directly proportional to the number of living cells in the well. All experiments were done in triplicates.

Cytotoxicity was measured using the MTT assay in the logarithmic phase of cell growth. Cells were incubated for 72 hours in 96-well microtiter plates before adding the nanoparticles. Fresh medium containing increasing concentrations of nanoparticles was added to each well and cells were incubated for another 48 hours. Ten µL of PBS containing 5 mg/mL MTT was dispensed to each well. The plates were incubated for two hours. Formazan was solubilized and measured as described above. The concentrations of materials were re-checked after completion of the experiments by atomic absorption spectroscopy of the authentic samples.

IC₅₀ values were calculated according to a four-parameter logistic equation. Data were plottet as a sigmoidal dose-response curve with variable slope using GraphPad PRISM software. For each material, the IC₅₀ values were determined from triplicate wells during the logarithmic cell growth phase. IC₅₀ values derived from logarithmic cell growth were routinely repeated in 3 independent experiments with almost identical results.

### DNA Microarray gene expression analysis

DNA microarray analysis was used to identify differentially expressed genes in untreated and AuNP treated HeLa cells. Total RNA was isolated using the Qiagen RNeasy kit. RNA quality was assessed using the RNA 6000 Nano Assay (Agilent Bioanalyser) and RNA quantity was estimated using the NanoDrop 1000. Total RNA was further processed according the GeneChip^{®} Whole Transcript (WT) Sense Target Labeling Assay Manual (Affymetrix, Santa Clara, CA, USA). The fragmented labeled sample was hybridized to an Affymetrix GeneChip^{®} Human Genome U133A 2.0 Array (Affymetrix). Experimental procedures for the Human Genome U133A 2.0 Arrays were performed according to the Affymetrix GeneChip® Expression Analysis Technical Manual. Briefly, total RNA (each 750 ng) was reverse transcribed into double-stranded cDNA using HPLC-purified T7-(dt) 24 primers and the GeneChip^{®} Expression 3-prime Amplification one-Cycle Target Labeling and Control Reagents-Kit. Subsequently, the purified double stranded cDNA was used as template to synthesize biotinylated complementary RNA probes. Hybridization to the DNA Array, containing 22,283 probesets representing approximately 14,500 well characterized human genes, was performed for 16 h at 45 °C and 60 rpm. After washing and staining the probe array using the Affymetrix Fluidics Station 450 the probe arrays were scanned using the Affymetrix GeneChip^{®} Scanner 3000. The microarray expression analysis was carried out using Bioconductor packages under R1 (see e.g. R. Gentleman, V. Carey, D. Bates, B. Bolstad, M. Dettling, S. Dudoit, B. Ellis, L. Gautier, Y. Ge, J. Gentry, K. Hornik, T. Hothorn, W. Huber, S. Iacus, R. Irizarry, F. Leisch, C. Li, M. Maechler, A. Rossini, G. Sawitzki, C. Smith, G. Smyth, L. Tierney, J. Yang, J. Zhang, Genome Biol 2004, 5, R80). Background correction and normalization were done with the GCRM algorithm A. MAS 5.0 (Affymetrix) was used for call detection.

### Cell Biology

**Flow cytometric determination of oxidative stress.** HeLa cells were plated in 6-well plates at initial densities of 40,000 in 2 mL grown for 72 hours. Fresh medium containing nanoparticles (100 µ1M Au1.4MS, 1000 µM Au1.1GSH and Au15MS) was added to the cells and incubated for 0, 6, 12, 18, 24, 48 hours. All cell-material combinations were set up in triplicates. After the AuNP incubation, cells were trypsinized and rinsed with PBS. After rinsing cells were suspended in a buffer containing 5-(and-6)-chloromethyl-2'7'-dichlorodihydrofluorescein diacetate acetyl ester (CM-H2DCFDA) (Molecuar Probes/Invitrogen). Cells were incubated for 30 minutes at 37 °C as described by the manufacturer. Shortly, 50 µg CM-H2DCFDA powder was dissolved in 100 µL ethanol as stock solution and was kept in -20 °C. For cell assays the stock solution was freshly diluted in PBS to a final working concentration of 2.5 µM. Cells incubated with 0.3 % H₂O₂ for 30 minutes served as positive control. 20,000 cells were analysed using FACSCalibur or FACSCanto flow cytometers and CELL-Quest software (Becton-Dickinson).

**Fluorescent mitochondria potential staining.** HeLa cells were plated in 96-well microtiter plates at initial densities of 2,000 in 100 µL and were incubated for 72 hours before adding nanoparticles. Fresh medium containing 100 µM Au1.4MS was added to each well and cells were incubated for an additional 0, 1, 6, 12, 18, 24 hours. All cell-material combinations were set up in triplicates. At the end of the incubation 100 µL JC-1 stain (Molecuar Probes/Invitrogen) at 2fold working concentration was added to each well and incubated for 30 minutes at 37 °C. Cells were rinsed, mounted in fresh PBS and analyzed by fluorescence microscopy. A stock solution of JC-1 was prepared in DMSO at 2.5 mg/mL and kept at -20 °C. The working concentration of JC-1 for HeLa cells was 3 µg/mL.

**Fluorogenic caspase activity determination.** HeLa cells were plated in 96-well microtiter plates at initial densities of 2,000 in 100 µL and were incubated for 72 hours before adding nanoparticles. Cells were rinsed and 30 µL of medium containing 50 µM Au1.4MS was added. Untreated cells served as negative control and cells treated with 0.2 µM staurosporine served as a positive control. Apoptosis was measured using the fluorogenic caspase substrate, Apo-ONE^{®} homogeneous caspase-3/7 assay (Promega) as described by the manufacturer. Briefly, Apo-ONE^{®} stock solution (100fold working concentration) was diluted 50fold in Apo-One^{®} buffer and 30 µL of this was added to each well and incubated at 23 °C for 3 hours. 50 µL of the clear supernatant was transferred to a black microtiter plate and measured using a Fluorostar plate fluorometer (BMG Labtech, Offenburg, Germany) at excitation wavelength 485 nm and emission wavelength 520 nm.

**Inhibition of apoptosis.** HeLa cells were plated in 96-well microtiter plates at initial densities of 2,000 in 100 µL and were incubated for 72 hours before adding reagents. Cells were left untreated or were pretreated with the caspase inhibitor Z-VAD-fmk for 3 hours (BACHEM N1510). A stock solution of 100 mM Z-VAD-fmk in DMSO was prepared and kept at -20 °C. Working solution was freshly prepared by diluting the stock solution in fresh cell culture medium.

### Inhibition of Au1.4MS cytotoxicity by antioxidant/reducing agent.

HeLa cells were plated in 96-well microtiter plates at initial densities of 2,000 in 100 µL and were incubated for 72 hours before adding reagents. Fresh medium containing nanoparticles and/or antioxidants (reducing agents) was added to each well and cells were incubated for another 48 hours. Final concentrations were NAC, 3 mM, GSH, TPPMS and ascorbic acid, 1 mM. Antioxidants/Reducing agents were freshly prepared by dissolving the powders in H₂O to 200 mM and further diluted in fresh cell culture medium to working concentration.

**Fluorescent cell proliferation assay.** HeLa cells were plated in 6-well plates and grown for 72 hours. Cell culture medium was removed and fresh medium containing the fluorescent cell staining dye, Carboxyfluorescein succinimidyl ester, CFSE (Invitrogen) for 30 minutes. Cells were rinsed with ice-cold PBS for 10 minutes to quench all background fluorescence. Fresh cell culture medium without or with 100 µM Au1.4MS was added and further incubated for 0, 1, 2, 3, 4 days. A stock solution of 10 mM in DMSO was kept in -20 °C. The stock solution was freshly diluted in PBS to 5 µM. 20,000 cells were analysed using FACSCalibur or FACSCanto flow cytometers and CELL-Quest software (Becton-Dickinson).

**Cell cycle and DNA content measurement using flow cytometry.** HeLa cells were plated in 6-well plates and incubated for 72 hours before adding nanoparticles. Fresh medium containing nanoparticles (100 µM Au1.4MS) was added to the cells were incubated for 0, 24 or 32 hours. All cell-material combinations were set up in triplicates. After the AuNP incubation, cells were trypsinized and rinsed with PBS. Washed cell pellets were fixed in chilled 70 % ethanol at 4 °C for 60 minutes. After fixation, cells were washed with PBS once again and resuspended in 250 µL PBS. RNAse (250 µL, 1 mg/mL) and propidium iodide (500 µL, 0.1 mg/mL) were added and incubated at room temperature for 15 minutes or overnight at 4 °C in the dark. Shortly before flow cytometry, cells were washed once with PBS. 20,000 cells were analysed using FACSCalibur or FACSCanto flow cytometers and CELL-Quest software (Becton-Dickinson).

## Claims

1. A gold nanocluster compound, especially a gold nanoparticle, having a reduced toxicity, especially cytotoxicity, preferably for use in medical diagnostics, said gold nanocluster compound comprising a core of at least one gold atom and at least one ligand bound to said core, wherein the reduction of toxicity, especially cytotoxicity, of said gold nanocluster compound is controlled and/or adjusted via its ligand structure and/or ligand chemistry.

2. The gold nanocluster compound of claim 1, wherein said gold nanocluster compound has a defined particle size, especially a defined size of the core of said gold nanocluster compound, said size ranging from 0.01 to 1,000 nm, especially from 0.1 to 100 nm, preferably from 0.5 to 50 nm, the outer limits of this range being included.

3. The gold nanocluster compound of claim 2, wherein the size of the core of said gold nanocluster compound ranges from 0.5 nm to 10 nm, especially 0.8 nm to 2 nm, preferably 1.0 nm to 1.5 nm, even more preferably 1.2 nm to 1.4 nm, the outer limits of these ranges being included, especially wherein the size of the core of said gold nanocluster compound is about 1.2 nm or about 1.4 nm.

4. The gold nanocluster compound of claim 2 or 3, wherein the size of the core of said gold nanocluster compound is at least 10 nm, especially at least 11 nm, preferably at least 12 nm.

5. The gold nanocluster compound of any of the preceding claims, wherein said gold nanocluster compound comprises gold atoms in the oxidation state of Au⁰ and/or wherein the core of said gold nanocluster compound comprises and/or consists of gold atoms in the oxidation state of Au⁰.

6. The gold nanocluster compound of any of the preceding claims, wherein said gold nanocluster compound comprises a core comprising from 5 to 200 gold atoms, especially 20 to 80 gold atoms, preferably 25 to 70 gold atoms, more preferably 30 to 60 gold atoms, even more preferably 35 to 55 gold atoms, the outer limits of these ranges being included and the gold being preferably in the oxidation state of Au⁰.

7. The gold nanocluster compound of any of the preceding claims, wherein the ligand structure and/or ligand chemistry is such that the gold nanocluster compound is stable under physiological conditions, especially if applied to a living organism, and/or that the bonding between said core and said ligand is not split or not cleaved under physiological conditions, especially not if applied to a living organism.

8. The gold nanocluster compound of any of the preceding claims, wherein said gold nanocluster compound has been treated with at least one antioxidant (reducing agent, antioxidative agent), especially wherein said reducing agent is preferably selected from the group consisting of N-acetylcysteine (NAC), glutathione (GSH), sulfonated triphenylphosphines, especially monosulfonated triphenylphosphines (TPPMS), and/or ascorbic acid as well as mixtures thereof.

9. The gold nanocluster compound of any of the preceding claims, wherein said gold nanocluster compound has been provided with a secondary ligand shell, especially wherein the secondary ligand surrounds and/or compasses said gold nanocluster compound including its core and its ligands and/or especially wherein the secondary ligand shell is provided to said gold nanocluster compound by an excess of said ligand.

10. The gold nanocluster compound of any of the preceding claims, wherein said gold nanocluster compound has been treated with at least one sulfur-containing organic compound, especially selected from the group consisting of thiols, dithiols, disulfides, thioethers, thioesters and mixtures thereof, preferably N-acetylcysteine (NAC) and/or glutathione (GSH) or mixtures thereof.

11. The gold nanocluster compound of any of the preceding claims, wherein said gold nanocluster compound comprises, as said ligands, organic ligands containing at least one sulfur atom for binding to the core of said gold nanocluster compound, preferable under formation of an Au-S-bonding, especially wherein the ligand is based on or derived from organic thiols, dithiols, disulfides, thioethers, thioesters and mixtures thereof, preferably N-acetylcysteine (NAC) and/or glutathione (GSH) or mixtures thereof.

12. The gold nanocluster compound of any of the preceding claims, wherein said gold nanocluster compound is represented by the general formula (I)
[Auₙ Lₘ] (I)
wherein:
• "Au" denotes the Au⁰ atoms in said gold nanocluster compound;
• "n" is a whole number denoting the number of gold atoms in said gold nanocluster compound, preferably n being selected in the range of from 20 to 80, especially 25 to 70, preferably 30 to 60, even more preferably 35 to 55, the outer limits of these ranges being included, with n being most preferably 35 if m = 35 or 55 if m = 12;
• "L", identical or different, denotes the ligand(s) in said gold nanocluster compound, especially an organic ligand, preferably a ligand based on a triphenylphosphine or a triphenylphosphine derivative, especially a sulfonated triphenylphosphine, a sulfonate, a sulfate, a phosphate, a phosphonate, a carbonate, a carboxylate or mixtures thereof, more preferably a ligand based on a triphenylphosphine or a triphenylphosphine derivative, especially a sulfonated triphenylphosphine;
• "m" is a whole number denoting the number of ligands in said gold nanocluster compound, preferably m being selected in the range of from 5 to 50, especially 10 to 40, preferably 12 to 35, the outer limits of these ranges being included, with m being most preferably 12 if n = 55 or 35 if n = 35,
wherein said gold nanocluster compound has been subjected to a stabilization treatment, wherein such stabilization treatment is performed:
• by treatment with at least one antioxidant, especially selected from the group consisting of N-acetylcysteine (NAC), glutathione (GSH), sulfonated triphenylphosphines, especially monosulfonated triphenylphosphines (TPPMS), and/or ascorbic acid as well as mixtures thereof; and/or
• by providing with a secondary ligand shell, especially wherein the secondary ligand surrounds and/or compasses said gold nanocluster compound including its core and its ligands and/or especially wherein the secondary ligand shell is provided to said gold nanocluster compound by an excess of said ligand; and/or
• by treatment with at least one sulfur-containing organic compound, especially selected from the group consisting of thiols, dithiols, disulfides, thioethers, thioesters and mixtures thereof, preferably N-acetylcysteine (NAC) and/or glutathione (GSH) or mixtures thereof, especially wherein the sulfur-containing organic compound binds via its sulfur atom(s) to the core of the gold nanocluster compound, preferable under formation of an Au-S-bonding.

13. A diagnostic composition, especially for the use in imaging procedures or techniques, especially in the field of human or veterinary medicine or in the field of material testing, preferably in the form of a liquid composition such as a solution or a dispersion, said diagnostic composition comprising at least one gold nanocluster compound as in any of Claims 1 to 12, preferably together with at least one essentially non-toxic carrier or excipient.

14. Use of at least one gold nanocluster compound as defined in any of Claims 1 to 12 in the fields of medicine, especially human and veterinary medicine, medical technology and material sciences, especially in the testing of materials.

15. Use of at least one gold nanocluster compound as defined in any of Claims 1 to 12 in the manufacture of a diagnostic composition, especially for use in imaging procedures or imaging techniques, particularly medical imaging such as X-ray imaging, computed tomography (CT) and photoacoustic imaging or imaging in material sciences such as testing of materials.

16. Use of at least one gold nanocluster compound as defined in any of Claims 1 to 12 as a contrast agent or a contrast enhancer in imaging procedures or imaging techniques, particularly medical imaging such as X-ray imaging, computed tomography (CT) or photoacoustic imaging or imaging in material sciences such as testing of materials.

17. A process of controlling and/or reducing the toxicity, especially cytotoxicity, of a gold nanocluster compound or gold nanoparticle, especially a ligand-stabilized gold nanocluster compound or gold nanoparticle, preferably for use in medical diagnostics, wherein the control and/or reduction of toxicity, especially cytotoxicity, of said gold nanocluster compound or gold nanoparticle is controlled and/or adjusted via its ligand structure and/or ligand chemistry, especially wherein said gold nanocluster compound or gold nanoparticle is as defined in any of Claims 1 to 12.

18. The gold nanocluster compound, the use and/or the process, each as defined in any of the preceding claims, wherein gold is at least partially replaced by another transition metal, especially selected from the group consisting of platinum (Pt), rhodium (Rh), iridium (Ir), palladium (Pd), ruthenium (Ru), osmium (Os) and silver (Ag) or mixtures thereof.
